# EUROPEAN PATENT APPLICATION

(11) **EP 1 338 886 A1**
(43) Date of publication of application: **27.08.2003**
(21) Application number: 03003571.1
(22) Date of filing: 17.02.2003
(51) Int. Cl.: G01N 1/28, B01L 3/14

(54) **Swab extraction system**

(30) Priority: 20.02.2002 US 81920
(71) Applicant: MAYO FOUNDATION FOR MEDICAL EDUCATION AND RESEARCH, Rochester, MN 55902 (US)
(72) Inventor: Uhl, James R., Rochester, Minnesota 55902 (US); Cockerill III, Franklin R., Rochester, Minnesota 55905 (US)
(74) Representative: Gassner, Wolfgang, Dr.

(57) **Abstract**

Disclosed are tools and techniques that can be used to rapidly and inexpensively obtain microorganisms from biological samples collected on swabs. The disclosed swab extraction systems and methods can speed diagnostic procedures and expedite therapeutic intervention, thereby improving patient outcomes.

## Description

### TECHNICAL FIELD

This invention relates to methods and materials for obtaining microorganisms from swab-collected biological samples.

### BACKGROUND

The first step of many medical diagnostic procedures involves the use of a swab to acquire a biological sample from a patient's mouth, throat, vagina, skin, or anus. In such procedures, microorganisms typically are mechanically transferred from the swab to a solid, semisolid or liquid growth medium for cultivation and subsequent analysis. Cultivation can be time intensive and can require specialized media, thereby delaying and increasing the cost of diagnosis.

### SUMMARY

The invention features tools and techniques that can be used to rapidly and inexpensively obtain microorganisms from biological samples collected on swabs. Use of the disclosed swab extraction systems can speed diagnostic procedures and expedite therapeutic intervention, thereby improving patient outcomes.

The invention features methods for collecting microorganisms from a swab sample. The methods involve centrifuging a swab extraction assembly. A swab extraction assembly has three components: 1) a swab sample including a swab and microorganisms; 2) an inner containment vessel having an open end sized so that the swab can be inserted into the inner containment vessel via its open end, and having an aperture through which the swab cannot be transmitted but through which the microorganisms can be transmitted; and 3) an outer containment vessel having an open end sized so that the inner containment vessel can be inserted into the outer containment vessel via its open end, and having a closed end so that when the outer containment vessel with a swab sample-containing inner containment vessel therein is centrifuged, the microorganisms are transmitted from the inner tube via the aperture and are retained by the outer containment vessel near the closed end. Microorganisms that are retained near the closed end of the outer containment vessel then can be recovered.

The featured methods can be used to recover bacteria, including, for example, Group A streptococcus (S. pyogenes), Corynebacterium diphtheriae, Neisseria gonorrhoeae, Mycoplasma pneumoniae, Bordetella pertussis, Chlamydia spp., Legionella spp., Mycobacterium spp., Staphylococcus aureus (nares), Herpes simplex, Varicella zoster virus, vancomycin resistant enterococci (VRE), Shigella spp., Salmonella spp., enterotoxigenic and hemorrhagic E. coli, Yersinia enterocolitica, Group B streptococcus (S. agalactiae), Vibrio spp., Aeromonas spp., Plesiomonas spp., Edwardsiella spp., Clostridium difficle, Chlamidia trachomatis, Candida albicans, Neisseria gonorrhoeae, Haemophilis ducrei, Gardnerella vaginalis, and Trichomas vaginalis.

The invention also features kits for obtaining microorganisms from a swab-collected biological sample. The kits include: 1) an inner containment vessel having an open end sized so that a swab can be inserted into the inner containment vessel via the open end of the inner containment vessel, and having an aperture through which a swab cannot be transmitted but through which microorganisms can be transmitted; and 2) an outer containment vessel having an open end sized so that the inner containment vessel can be inserted into the outer containment vessel via the open end of the outer containment vessel, and having a closed end so that when the outer containment vessel with a swab sample-containing inner containment vessel therein is centrifuged, microorganisms are transmitted from the inner tube via the aperture and are retained by the outer containment vessel near the closed end. Either or both the inner containment vessel and outer containment vessel can be sterile. Either or both the inner containment vessel and outer containment vessel can be free of nucleases. Kits also can include any or all of the following: packaging material (e.g., with instructions indicating that the kit is effective for obtaining microorganisms from a swab-collected biological sample); a swab for collecting a biological sample; and a diluent for a swab.

Other features and advantages of the invention will be apparent from the following detailed description, and from the claims.

Unless otherwise defined, all technical and scientific terms used herein have the meaning commonly understood by one of ordinary skill in the field of this invention. All publications, patent applications, patents and other references mentioned herein are incorporated by reference in their entirety. In case of conflict, the present specification, including definitions, will control. The disclosed materials, methods, and examples are illustrative and are not intended to be limiting. Skilled artisans will appreciate that methods and materials similar or equivalent to those described herein can be used to practice the invention.

### DESCRIPTION OF DRAWINGS

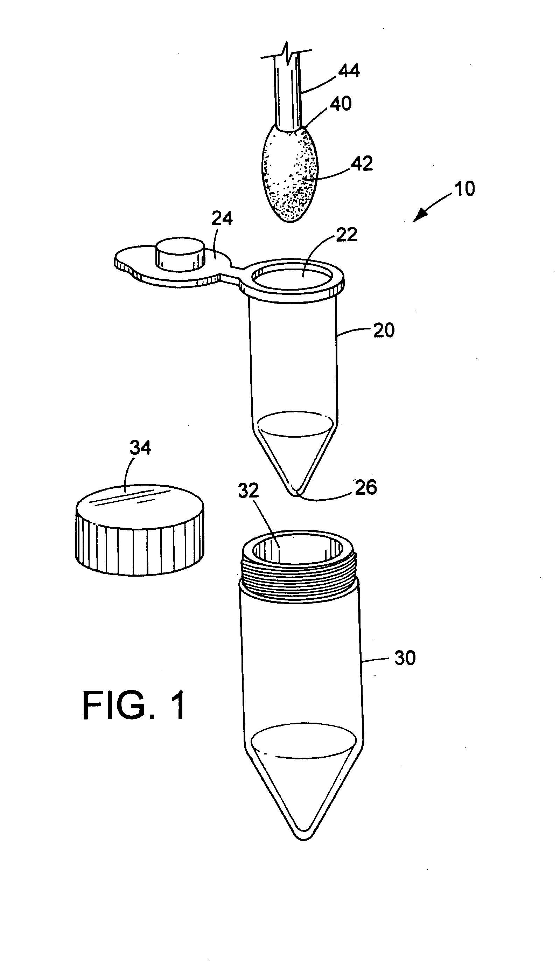

FIG. 1 is a schematic diagram of a swab extraction system.

### DETAILED DESCRIPTION

The invention provides swab extraction systems and methods for obtaining microorganisms (e.g., bacteria and viruses) from biological specimens on swabs. Recovered microorganisms can be used for subsequent diagnostic procedures, including nucleic acid, immunological, and physiological analysis.

### Swab Extraction Systems

A swab extraction system ("SES") 10 includes two containment vessels, an inner swab containment vessel ("ISCV") 20 and an outer cell containment vessel ("OSCV") 30. See Figure 1.

An ISCV 20 has an open end 22 sized so that the sampling portion 42 of a swab 40 can be inserted into the ISCV through the open end 22. An ISCV 20 can have an attached cap 24 (e.g., a snap-cap) that can cover the open end 22. The open end 22 of an ISCV 20 also can be covered by a detachable cap (e.g., a screw-cap). A swab extraction system 10 can include a detachable cap for an ISCV. A film such as Parafilm M® (Structure Probe, Inc.) also can be used to cover the open end 22 of an ISCV 20. A swab extraction system 10 can include a film suitable for covering the open end 22 of an ISCV 20. In some embodiments, an ISCV 20 does not have a cap and is not covered.

An ISCV 20 has at least one aperture 26 through which microorganisms can be transmitted, but through which the collection end 42 of a swab cannot be wholly transmitted during centrifugation at 20,000 x g. An aperture 26 typically spans between about 0.1 mm to 3.0 mm, but need not be uniform in diameter. An aperture 26 can be formed in a vessel using a sharp, pointed instrument (e.g., an awl), a boring instrument (e.g., a drill bit), or a sharp blade. Vessels suitable for such manipulations include 0.2 to 1.0 ml capacity microfuge tubes (e.g., Fisherbrand Cat. No. 05-408-16, Fisherbrand Cat. No. 02-681-311, Eppendorf Cat. No. 22 60 000-1, and Costar Cat. No. 3208). An ISCV 20 also can be fashioned from a tapered cylinder (e.g., a pipette tip) by one of skill in the art. An ISCV 20 also can be made by injection molding a polymeric substance using a mold and process that yields a vessel having an appropriately sized open end and an appropriately sized aperture.

An OSCV 30 has an open end 32 sized so that an ISCV 20 can be inserted into the OSCV 30 through the open end 32. An OSCV 30 can have an attached cap (e.g., a snap-cap) that can cover the open end 32. The open end of an OSCV also can be covered by a detachable cap 34 (e.g., a screw-cap). A swab extraction system can include a detachable cap 34 for an OSCV 30. A film such as Parafilm M® (Structure Probe, Inc.) also can be used to cover the open end 32 of an OSCV 30. A swab extraction system can include a film suitable for covering the open end 32 of an OSCV 30. In some embodiments, an OSCV 30 does not have a cap and is not covered.

An OSCV 30 is sized to be accommodated by a centrifuge rotor, and an ISCV 20 is sized to be inserted into an OSCV 30. In some applications, a swab extraction system 10 is centrifuged in a microcentrifuge, such as an Eppendorf MiniSpin Personal Micro Centrifuge (Cat. No. 22 62 010-0), or Eppendorf 5417C (Cat. No. 22 62 170-0), Eppendorf 5415D (Cat. No. 22 62 121-1), Eppendorf 5417R (Cat. No. 22 62 140-7), of Eppendorf 5415R (Cat. No. 22 62 140-8). Typically, microcentrifuges have rotors (e.g. Eppendorf Cat. Nos. 22 63 600-6, 22 63 605-7, 22 63 612-0, 22 63 613-8, and 22 63 614-6) that, with adaptors, can accommodate microcentrifuge tubes having a capacity between 0.2 ml and 2.0 ml.

In one embodiment, an OSCV has a capacity of about 1.5 to 2.0 ml (e.g., Fisherbrand Cat. No. 05-408-10, Fisherbrand Cat. No. 02-681-320, Eppendorf Cat. No. 22 60 002-8, and Costar Cat. No. 3620), and an ISCV has a capacity of about 0.5 to 0.7 ml. ISCVs and OSCVs typically are made of a polymeric substance (e.g., polypropylene, polyethylene, and polystyrene). ISCVs and OSCVs can be made free of any or all of the following prior to assembly and use: microorganisms, nucleic acids, and inhibitors of nucleic acid analyses (e.g., nucleases), such as the polymerase chain reaction (PCR).

In another embodiment, an OSCV (e.g., a 2 ml Eppendorf tube) can contain 10 to 400 71 of ceramic, silica zirconium, or silica/zirconium type beads. The beads are typically about 0.1 mm in diameter, but can be larger or smaller in size.

A swab extraction system 10 can include a swab 40. A swab has a sampling portion 42 and a shaft 44. A shaft 44 typically is made of a flexible polymer (e.g., plastic), and a sampling portion 42 typically is made of a fibrous material (e.g., Dacron, Rayon, and cotton). Swabs can be obtained commercially from, for example, Copan Diagnostics, Inc. (Corona, CA, U.S.A.) (e.g., Cat. Nos. 140C, 141C, 155C, 166C, and 159C), and from Starplex Scientific, Inc (Etobicoke, ON, Canada) (e.g., Cat. Nos. S09, SP130X, and SP132).

### Swab Extraction Methods

Swab extraction systems can be used to obtain microorganisms such as Group A streptococcus (*S*. *pyogenes*), *Corynebacterium diphtheriae, Neisseria gonorrhoeae, Mycoplasma pneumoniae, Bordetella pertussis, Chlamydia* spp., *Legionella* spp., and *Mycobacterium* spp. from a patient's throat. Swab extraction systems can be used to obtain microorganisms such as *Staphylococcus aureus* (nares), Herpes simplex, Varicella zoster virus, and Group A streptococcus (*S. pyogenes*) from a patient's skin. Swab exraction systems can be used to obtain organisms such as vancomycin resistant enterococci (VRE), *Shigella* spp., *Salmonella* spp., enterotoxigenic and hemorrhagic *E. coli, Yersinia enterocolitica,* Group B streptococcus (S. agalactiae), *Vibrio* spp., *Aeromonas* spp., *Plesiomonas* spp., *Edwardsiella* spp., and *Clostridium difficle* from a patient's anus. Swab extraction systems can be used to obtain organisms such as *Chlamidia trachomatis, Candida albicans, Neisseria gonorrhoeae, Haemophilis ducrei, Gardnerella vaginalis, Trichomas vaginalis,* and Group B streptooccus (*S. agalactiae*) from a patient's vagina.

The sampling portion 42 of a swab 40 used to acquire a biological sample is hydrated with a diluent such as Cary-Blair medium, Stuart's medium, Amie's medium, buffered glycerol saline, or water. A suitable diluent often is supplied by a swab supplier, but can be included in a swab extraction system 20. The hydrated sampling portion 42 of a swab 40 is inserted into an ISCV 20 through the open end 20. The shaft 44 of a swab 40 can be broken or cut if it would otherwise interfere with covering the open end 22 of an ISCV 20 or the open end 32 of an OSCV 30, or with the operation of a centrifuge into which a swab extraction system 10 is placed. To facilitate transfer of a swab sampling portion 42 to an ISCV 20, a swab having a snap-off sampling portion 42 can be used.

An swab extraction system assembly is made such that an ISCV 20 having a hydrated sampling portion 42 of a swab 40 inserted therein is nested within an OSCV 30. The swab extraction system assembly is placed into a centrifuge rotor and is centrifuged (e.g. at 20,000 x g for 3 minutes) so that fluid and microorganisms are transmitted from the sampling portion 42 through an aperture 26 of the ISCV 20 into the OSCV 30. The ISCV 20 having a sampling portion 42 of a swab 40 therein is removed from the OSCV 30.

Fluid collected in the OSCV 30 can be analyzed, and microorganisms adhering to the inside of the OSCV can be analyzed. In some applications, fluid collected in an OSCV 30 is discarded and microorganisms adhering to the inside of the OSCV 30 are resuspended in water or a buffer appropriate for subsequent analysis or culturing. In applications where recovered microorganisms are cultured, the components of the swab extraction system typically are sterilized prior to use. In applications where recovered microorganisms are subjected to nucleic acid analyses, resuspended microorganisms typically are processed to expose nucleic acids for analysis (e.g., by boiling).

### Swab Extraction Kits

Elements of a swab extraction system 10 can be combined with packaging material and sold as a kit for obtaining microorganisms from swab-collected biological samples. Thus a kit can include an ISCV 20 and an OSCV 30. A kit also can include any or all of the following: a covering ISCV 20,or an OSCV, a swab 40, a diluent suitable for hydrating a swab 40, an object or material for covering the open end 22 of an ISCV 20 or the open end 32 of an OSCV 30. The packaging material included in a kit typically contains instructions or a label describing how the various elements of a swab extraction system 10 are effective for obtaining microorganisms from swab-collected biological samples. Components and methods for producing such kits are well known.

The invention will be further described in the following examples, which do not limit the scope of the invention described in the claims.

### EXAMPLES

### Example 1: Extraction of Streptococcus pyogenes from a throat swab.

This example demonstrates the use of a swab extraction system to extract *Streptococcus pyogenes* from a throat swab.

A swab extraction system was prepared by punching a hole in the bottom of a 0.65 ml snap-cap centrifuge tube (Intermountain Scientific Corporation, Cat. No. C-3300-2) with an awl, and placing the punctured tube within a 2 ml screw-cap centrifuge tube (Sarstedt Cat. No. 72.693.005).

A rayon swab transported with Stuarts medium (Copan, Cat. No. 41C) was used to collect a sample from a patient's throat. The handle of the swab was covered with a Bio-Screen® Biohazard wipe (Fisher Cat. No. 14-412-52C), and broken off so that the sample collecting portion of the swab fit within the 0.65 ml centrifuge tube. The snap-cap lid was closed.

The swab extraction assembly was centrifuged at 20,800 x *g* for 3 minutes in an Eppendorf 5741C centrifuge to extract microorganisms and medium from the swab into bottom of the 2 ml centrifuge tube. The 0.65 ml centrifuge tube and swab were discarded. The supernatant was carefully removed from the 2 ml centrifuge tube with a fine-tip transfer pipette and was discarded. The inside surface (sides and bottom) of the 2 ml tube was washed with 100 µl of water to suspend recovered bacterial cells. The 2 ml centrifuge tube was capped and placed in a 100 °C heating block for 10 minutes. The tube was then centrifuged at 20,800 x *g* for 3 minutes. Recovery of *Streptococcus pyogenes* was determined by nucleic acid analysis of the supernatant.

From a sample processed in parallel, *Streptococcus pyogenes* was cultured from cellular material extracted the first centrifugation step. For this sample, the inside surface (sides and bottom) of the 2 ml tube was washed with 100 µl of serile saline to suspend recovered bacterial cells.

### Example 2: Extraction of Herpes simplex virus from a dermal swab.

This example demonstrates the use of a swab extraction system to extract Herpes simplex virus from a dermal swab.

A swab extraction system was prepared by punching a hole in the bottom of a 0.65 ml snap-cap centrifuge tube (Intermountain Scientific Corporation, Cat. No. C-3300-2) with an awl, and placing the punctured tube within a 2 ml screw-cap centrifuge tube (Sarstedt, Cat. No. 72.693.005).

A rayon swab transported with Stuarts media (Copan, Cat. No. 141C) was used to collect a sample from a patient's skin lesion. The handle of the swab was covered with a Bio-Screen® Biohazard wipe (Fisher, Cat. No. 14-412-52C), and broken off so that the sample collecting portion of the swab fit within the 0.65 ml centrifuge tube. The snap-cap lid was closed.

The swab extraction assembly was then centrifuged at 20,800 x g for 3 minutes in an Eppendorf 5741C centrifuge to extract cells and media from the swab into bottom of the 2 ml centrifuge tube. The 0.65 ml centrifuge tube and swab were discarded. Recovery of Herpes simplex virus was determined by nucleic acid analysis of the supernatant.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A method for collecting microorganisms from a swab sample, comprising the steps:
a) centrifuging a swab extraction assembly comprising:
i) a swab sample comprising a swab and said microorganisms,
ii) an inner containment vessel having an open end sized so that said swab can be inserted into said inner containment vessel via said open end of said inner containment vessel, and having an aperture through which said swab cannot be transmitted but through which said microorganisms can be transmitted, and
iii) an outer containment vessel having an open end sized so that said inner containment vessel can be inserted into said outer containment vessel via said open end of said outer containment vessel, and having a closed end so that when said outer containment vessel with a swab sample-containing inner containment vessel therein is centrifuged, said microorganisms are transmitted from said inner tube via said aperture and retained by said outer containment vessel near said closed end, and
b) collecting microorganisms that are retained near said closed end of said outer containment vessel.

2. The method of claim 1, wherein said microorganisms are bacteria.

3. The method of claim 1 wherein said microorganisms are selected from the group consisting of: Group A streptococcus (*S. pyogenes*), *Corynebacterium diphtheriae, Neisseria gonorrhoeae, Mycoplasma pneumoniae, Bordetella pertussis, Chlamydia* spp., *Legionella* spp., *Mycobacterium* spp., *Staphylococcus aureus* (nares), Herpes simplex, Varicella zoster virus, vancomycin resistant enterococci (VRE), *Shigella* spp., *Salmonella* spp., enterotoxigenic and hemorrhagic *E. coli, Yersinia enterocolitica*, Group B streptococcus (S. agalactiae), *Vibrio* spp., *Aeromonas spp., Plesiomonas spp., Edwardsiella spp., Clostridium difficle, Chlamidia trachomatis, Candida albicans, Neisseria gonorrhoeae, Haemophilis ducrei, Gardnerella vaginalis,* and *Trichomas vaginalis.*

4. A kit for obtaining microorganisms from a swab-collected biological sample, said kit comprising:
a) an inner containment vessel having an open end sized so that a swab can be inserted into said inner containment vessel via said open end of said inner containment vessel, and having an aperture through which said swab cannot be transmitted but through which said microorganisms can be transmitted, and
b) an outer containment vessel having an open end sized so that said inner containment vessel can be inserted into said outer containment vessel via said open end of said outer containment vessel, and having a closed end so that when said outer containment vessel with a swab sample-containing inner containment vessel therein is centrifuged, microorganisms are transmitted from said inner tube via said aperture and are retained by said outer containment vessel near said closed end, and
c) packaging material.

5. The kit of claim 4, wherein said packaging material indicates that said kit is effective for obtaining microorganisms from a sample.

6. The kit of claim 4, wherein said inner containment and said outer containment vessel are sterile and free of nucleases.

7. The kit of claim 4, further comprising a swab diluent.

8. The kit of claim 4, further comprising a swab for collecting a biological sample.
